# EUROPEAN PATENT SPECIFICATION

(11) **EP 2 159 535 B1**
(45) Date of publication and mention of the grant of the patent: **18.08.2010**
(21) Application number: 08015180.6
(22) Date of filing: 28.08.2008
(51) Int. Cl.: G01B 9/02, A61B 3/12, G01N 21/47

(54) **Apparatus for optical coherence tomography and non-interferometric imaging**
Vorrichtung zur optischen Kohärenztomographie und nichtinterferometrischen Abbildung
Appareil pour tomographie de cohérence optique et imagerie non interférométrique

(43) Date of publication of application: 03.03.2010
(73) Proprietor: Optopol Technology S.A., 42-400 Zawiercie (PL)
(72) Inventor: Bajraszewski, Tomasz, 87-100 Torun (PL); Szkulmowski, Maciej, 87-100 Torun (PL); Dalasinski, Pawel, 87-100 Torun (PL); Wojtkowski, Maciej, 87-851 Boniewo (PL)
(74) Representative: Glawe, Delfs, Moll

(56) References cited:
- WO-A-2007/130411
- US-A1- 2006 058 682
- I. TRIFANOV, M. HUGHES, A.GH. PODOLEANU, R.B. ROSEN: "Quasi-simultaneous optical coherence tomography and confocal imaging" JOURNAL OF BIOMEDICAL OPTICS, vol. 13, no. 4, 18 July 2008 (2008-07-18), pages 044015-1-044015-7, XP007906560
- A.G. PODOLEANU: "Combining SLO and OCT technology" BULL. SOC. BELGE OPHTALMOL., vol. 302, 2006, pages 133-151, XP007906559
- M. PIRCHER, B. BAUMANN, E. GÖTZINGER, C.K. HITZENBERGER: "Retinal cone mosaic imaged with transverse scanning optical coherence tomography" OPTICS LETTERS, OSA, OPTICAL SOCIETY OF AMERICA, WASHINGTON, DC, US, vol. 31, no. 12, 15 June 2006 (2006-06-15), pages 1821-1823, XP001243248 ISSN: 0146-9592

## Description

The invention relates to an apparatus for optical coherence tomography and non-interferometric imaging of a sample.

Optical coherence tomography (OCT) is a technique for examination of three-dimensional structures of partially transparent matter. According to this technique, partially coherent light is divided into two portions. One portion is used to illuminate the sample under investigation. The second portion is led through a reference path to be recombined with the light back-scattered from the sample. The recombined light contains an interferometric signal containing information about the internal structure of the sample. This information can be retrieved in essentially two different ways.

The first way, known as "Time domain Optical Coherence Tomography" (TdOCT), is based on a scannable optical path delay introduced in the reference path. The delay is scanned in an oscillating manner. In this case, interference fringes occur only in certain scan positions, namely in positions in which the optical path length of the reference path is identical with the optical path length for the light back-scattered by the sample. This enables determination of the relative distances of back-scattering structures within the sample (see Huang et al., Science, Vol. 254, 1991, p. 1178 to 1181).

The second way for retrieving information from the recombined light, known as "Fourier domain Optical Coherence Tomography" (FdOCT) is based on spectral analysis of the recombined light. The spectrum of the recombined light, i.e. the distribution of the light intensities for the various spectral components, is recorded. This can be performed by using either a spectrometer ("Spectral Optical Coherence Tomography", SOCT; see Szkulmowska et al., Journal of Physics D: Applied Physics, Vo. 38, 2005, 2606 - 2611), or a tuned light source ("Swept Source Optical Coherence Tomography", SS-OCT; see R. Huber et al., Optics Express, Vo. 13, 2005, 3513 - 3528).

A general problem associated with OCT is to locate the region of which a microscopic 3D image is recorded within the macroscopic structure. If, for instance, OCT is performed on the retina of the human eye, the operator must be enabled to match the automatically generated 3D with a particular area of the retina. To this end, hybrid devices have been proposed generating both OCT images and non-interferometric images of the same sample in parallel. In order to attain sufficient matching of the OCT and non-interferometric images, the imaging systems share the same lens system for illuminating the eye and collecting the light returning from the eye.

Hybrid devices have been proposed in which the same detector is used for OCT and non-interferometric imaging (WO 2007/130411 A2; Trifanov et al., Quasi-simultaneous optical coherence tomography and confocal imaging, J. Biomed. Opt., vol. 13 (4), p. 044015-1 - 044015-7). Such solutions suffer from the drawback that OCT and non-interferometric imaging require different detector characteristics for optimal function.

For this reason, hybrid devices with separate detectors for OCT and non-interferometric imaging are preferred. For example, SOCT requires a detector with a dispersive element, e.g. a grating. TdOCT is preferably performed with a combination of two photodetectors with one balanced photoreceiver to obtain the interference fringes at a high signal-to-noise ratio, whereas fast and sensitive detectors such as avalanche photodiodes (APD) or photomultipliers are preferred for non-interferometric images. Two separate detectors require the light returning from the eye to be split into two portions, one for each detector.

The splitting elements in most prior art hybrid devices are bulky elements such as thick plate beam splitters positioned in the path of the light returning from the eye (WO 2008/052311 A1, US 2007/0291277 A1). Placing the splitting elements in the path of the recombined light allows more compact designs, in particular, if optical fiber components are employed (WO 2008/052311 A1). But at the same time, better signal-to-noise ratios are obtained if the splitting is performed with the light returning from the sample prior to recombination with reference light (WO 2008/052311 A1).

The object of the invention is to provide a design for a hybrid device which facilitates a compact design without a reduction of the signal-to-noise ratio. It achieves this result by the elements of claim 1. The dependent claims describe further improvements of the invention.

As far as the term light is used with reference to the invention, it means electro-magnetic radiation within a wavelength range of 600 nm to 1,700 nm. The terms optical and optics within the meaning of the invention denote that the respective elements or steps influence such radiation.

Non-interferometric imaging within the meaning of the invention comprises any kind of optical image formation. The invention is particularly useful confocal image formation (e.g. US 3,013,467), as this technique may share the same scanning optics with OCT. The invention is particularly advantageous in ophthalmology, where the identification of the region examined with OCT in the eye is very difficult. For these cases, the non-interferometric imaging unit is preferably a laser scanning ophthalmoscope.

The sample is, of course, not a part of the apparatus of the invention. However, the apparatus has a position where the sample is to be placed for proper operation. Generally, if the invention is used in human or veterinary medicine, the result obtained from the use of the invention may help, possibly together with other facts known about the patient or animal, to make a diagnosis.

The apparatus according to the invention may comprise optical elements for focusing and other optical adjustment, such as polarization controllers, neutral density filters, means for compensating unwanted dispersion effects, as known to the person skilled in the art. The apparatus may further comprise scanning optics configured for scanning the beam incident on the sample transversally, i.e. in one or two directions perpendicular to the axis of the beam incident on the sample.

Illuminating optics within the meaning of the invention include at least one light source, preferably a single light source for simplicity. Preferred light sources are semiconductor light sources, i.e. types in which the radiation is generated by a semiconductor, such as, but not limited to, light emitting diodes, laser diodes. Such light sources offer good stability combined with a small size. Laser diodes may be operated below laser threshold to obtain the required coherence characteristics. Especially preferred as one of the light sources or as the single light source are superluminescent diodes (SLD) due to their benign coherence properties for OCT. At least one light source is preferably operated in a mode where the spatial coherence is optimized for OCT imaging. The illuminating optics may comprise additional light sources, in particular light source especially adapted for non-interferometric imaging, e.g. a laser or laser diode for confocal laser scanning microscopy. One of the light sources may be adapted for fluorescence imaging to add to the versatility of the apparatus.

In the case of multiple light sources, suitable optics for coupling in the light from the additional light sources in the path of the light from the first light source will be provided as known to the person skilled in the art. Non-reciprocal optical elements such as optical insulators may be part of the illumination optics to prevent returning light from entering the light source or light sources. Finally, the illuminating optics may comprise means for directing the light from the light source or light sources, respectively, to the sample.

The illuminating optics preferably comprises optical fibers. More preferably, it comprises one optical fiber extending from the light source to a point where the light is coupled out for focusing on the sample and transversal scanning, if applicable. In the case of multiple light sources, connecting lines branch off from the line to the additional light source or sources. These connecting lines preferably also consist of optical fibers, and fiber optic couplers are provided for coupling in the light from the additional light source or sources. The optical fibers of the illuminating optics thus have a single line or, in the case of two or more light sources, tree design. This extensive use of optical fibers for the illuminating path facilitates a compact design of the apparatus according to the invention. For the same reason, optical fiber connectors are provided as interfaces for at least one light source, preferably all light sources, with the optical fibers. So the use of space-consuming bulk optics may be reduced to the lowest possible extent.

The illumination path extends from the light source to the position of the sample. If more than one light sources are used, the illumination path extends by definition from the light source which is arranged for OCT, i.e. which generates light with the required coherence. Should more than one light source be used for OCT, the illumination path extends by definition from that OCT light source with the geometrically shortest distance to the sample.

The first splitting optics preferably comprises an optical coupler connected to the optical fiber of the illumination path.

Likewise, the recombining optics preferably comprises a fiber optics coupler. It may also be connected to the optical fiber of the illumination path. Advantageously, the recombining optics is identical with the first splitting optics, i.e. the splitting and the recombination are effected by the same physical component or components, as is the case with Michelson interferometers. This variant of the invention requires less optical components which helps achieving compactness.

Furthermore, the second splitting optics preferably comprises a fiber optics coupler. It may also be connected to the optical fiber of the illumination path. Advantageously, the second splitting optics is identical with the recombining optics, i.e. the splitting and the recombination are effected by the same physical component or components.

It is highly advantageous if the first splitting optics, the recombining optics and the second splitting optics are identical, i.e. each splitting and the recombination are performed by the same physical component. This variant of the invention reduces the number of optical components to a minimum and thus facilitates an extremely compact design. Even if bulk optics are used for the combined splitting and recombination device, less space and alignment than in prior art designs is required as one component performs multiple functions at once.

The output path extends from the recombining optics to the optical coherence tomography detection device. Because the recombining optics can be identical with the second splitting optics, as indicated above, it becomes clear that the recombining optics itself is disposed along the output path by definition.

An optical coherence detection device within the context of the invention is a device which is configured to convert the optical signal into an electric signal containing interferometric pattern of the optical signal. The optical coherence detection device is preferably adapted for the particular OCT method, typically either TdOCT, SOCT or SS-OCT. In the case of SOCT, the optical coherence detection device may comprise dispersion means, e.g. a grating or prism, for dispersion of the detected light into spectral components. Furthermore, it may comprise an array of detector cells, e.g. a CCD, InGaAs or CMOS array, disposed in such a way, that each cell receives a certain spectral portion of the dispersed light.

A processing unit arranged for evaluating the interferometric pattern in the electric signal may be part of the apparatus according to the invention. In the case of FdOCT, such processing unit may comprise means for carrying out a Fourier transform, in particular a Fast Fourier Transform (FFT), to obtain the locations of backscattering surfaces within the sample from the interferometric pattern. In the case of SOCT, such processing unit may additionally comprise means for converting an I(λ) spectrum (light intensity as a function of wavelength) into an I(k) spectrum (light intensity as a function of wavenumber), e.g. by interpolation, as an I(k) spectrum is more suitable for a Fourier transform to a function of geometric or optical distances as desired in OCT.

Within the context of the invention, the term interrupting encompasses any kind of preventing the light in the reference path from reaching the recombining optics. This may also comprise an attenuation of that reference light to a degree that it does not substantially impair the operability of the second detection device. However, a complete interruption, e.g. by fully blocking or deflecting from the reference path, is preferred to ensure proper functioning.

Advantageously, the interrupting means is provided by a scannable optical delay line. Many OCT applications, such as TdOCT in general and SOCT where phase-shifts are applied between recordings of spectra, require scannable optical delay lines (ODL) within the reference path anyway. If one or more of the components of such an ODL perform the interrupting, no additional parts are needed which, again, contributes to the compactness of the entire apparatus. So the advantages of the invention become particularly prominent for those types of OCT devices which are equipped with a scannable ODL.

More advantageously, the scannable ODL line comprises a pivotable mirror arranged for deflecting light to a second mirror arranged for directing the light along the reference path to the recombining device. Such an ODL offers a very high scanning speed, which is of particular advantage for the invention as a high scanning speed allows close interleaving of the imaging formation with the two or more detectors and, therefore, good matching between the OCT and the non-interferometric image. An example for such an ODL is described by Liu et al., Optics Letters, Vol. 29, 2004, p. 80 - 82, where an intermediate curved mirror is placed between the pivotable and the second mirror. A pivotable mirror within this context can be a rotatable mirror as well. Polygonal mirrors may be used.

Further advantageously, the pivotable mirror is arranged for pivoting into an angle position in which the deflected light does not reach the reflecting surface of the second mirror. In this manner, a very simple but effective interrupting means is assembled with the only measure of enlarging the pivoting range of the pivotable mirror. Alternatively, the pivotable mirror may be arranged in such a way that the deflected light does reach the second mirror, but in a position and angle in which it is prevented from travelling further along the reference path to the recombining device. However, such an arrangement is more complicate.

Advantageously, the apparatus according to the invention comprises a triggering device activating the second detection device in phases in which the light in the reference path is interrupted. Such a device helps to ensure that the second detection device comes only in operation when the reference light is interrupted and thus cannot spoil the signal-to-noise ratio. This triggering device may be arranged either to receive a signal from the interrupting means or to trigger the interrupting means as well.

Advantageously, the first and second splitting optics and the recombining optics are optical couplers. This allows for a compact design. For the same reason, it is further advantageous, the connections between these optics consist of optical fibers. All optical fibers connected to the said optics are preferably single-clad fibers in order to keep the cheap and comparatively easy to align. Preferably, such optical fibers are used in the broadest possible extent. That means that all present light sources are as well as the second detecting device are directly connected to the optical fibers. The same applies to the interrupting means if the reference path consists of optical fibers. In TdOCT or SS-OCT the detector may also be directly connected to the optical fiber system. Ordinary SOCT detectors require bulk optics for the dispersion, but one may employ an arrayed waveguide grating as dispersive element as well and in this case direct connection to the optical fiber system is preferred.

Some embodiments of the invention as examples will now be described in greater detail with reference to drawings, in which:

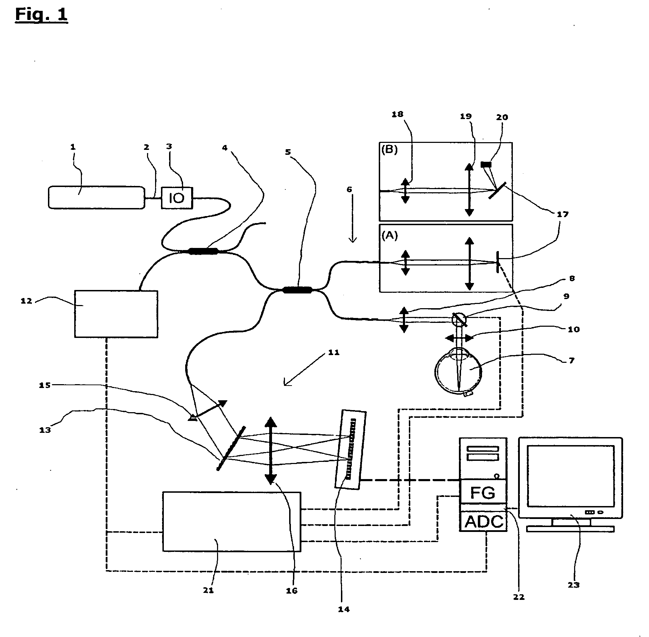

- Fig. 1: is a schematic block diagram of an apparatus for optical coherence tomography and non-interferometric imaging according to the invention;
- Fig. 2: is a schematic block diagram of a first alternative embodiment of the terminal part of the reference path of an apparatus according to the invention;
- Fig. 3: is a schematic block diagram of a second alternative embodiment of said terminal part;
- Fig. 4: is a schematic block diagram of a third alternative embodiment of said terminal part;
- Fig. 5a, 5b, 5c: are schematic block diagrams of a fourth alternative embodiment of said terminal part constituting a scannable ODL in three scanning positions.

Coherent broadband light is emitted by a light source 1 into an optical fiber 2 which is directly connected to the light source, e.g. a SLD. An optical insulator 3 is placed downstream in order to prevent returning light from entering damaging the light source 1. The light passes a coupler 4, the function of which becomes apparent when the returning light is discussed.

Coupler 4 forwards the light from the light source further to another coupler 5. Coupler 5, acting as first splitting optics, splits the light into a reference portion entering the reference path 6 and a sample portion for illuminating the sample, which is represented by a symbolized eye 7 here. The sample portion exits the optical fiber 2, is collimated by a collimating lens 8 and directed to scanning optics which is represented by a galvanometric scanner mirror 9 here. A focusing lens 10 focuses the sample beam on the region of the eye 7 under examination.

The backscattered light returns the same way, i.e. from the eye 7 through focusing lens 10, now acting as collimating lens, to the scanning optics 9 which descans the returning light, and through collimating lens 8, now focusing the beam on the end of the optical fiber 2. The returning light propagates to coupler 5, which recombines it with the returning reference portion, i.e. coupler 5 acts as recombining optics.

Moreover, the same coupler 5 acts as second splitting device splitting the recombined light immediately after recombination into two portions, one of which proceeding to a spectrometer 11 for OCT detection. The other portion is directed back to coupler 4 where one portion of it is split off to propagate to a detector 12 for non-interferometric imaging which is preferably an APD directly connected to the optical fiber leading from coupler 4 to detector 12. The optical insulator 3 prevents the other portion split off by coupler 4 from entering the light source 1.

The coupling ratio of coupler 4 is preferably 70/30 so that 30 % of the light fed by the light source propagates to the other coupler 5, while 70 % of the light returning from that coupler 5 is directed to the detector 12. If the coupling ratio of the other coupler 5 is, as preferred, 50/50, about 35 % of the light returning from the eye 7 reaches the detector 12. This suffices for practical applications, especially if an APD is employed as detector 12.

Other coupling ratios for both couplers 4 and 5 may be used, particularly between 60/40 and 80/20 for coupler 4 and between 30/70 and 70/30 for coupler 5.

It is to be noted that coupler 5 acts as first splitting optics, recombining optics and second splitting optics, so one and the same element performs three functions at once. This allocation of three functions to a single component allows a compact design in an elegant manner. The combination of optical insulator 3 and coupler 4 simply serves to divert returning light to the light source 1. Said combination may be replaced, for instance, by an optical circulator allowing all light emerging from the light source to pass on to coupler 5, but directing all returning light to detector 12. Both alternatives allow the output path to coincide in part, namely a part connected to the second splitting optics, with the illumination path. This, again, contributes to the compactness. Non-reciprocal optical devices, such as optical circulators or optical insulators, are used to shield the light source from returning light. Furthermore, a standard device, such as a 2 x 2 coupler or standard beam splitter, can be employed to perform the tasks of the first splitting optics, recombining optics and second splitting optics.

The spectrometer 11 separates the spectral components of the light with a grating 13. The spectral components are directed to a line sensor 14, e.g. a CCD array. A collimating lens 15 collimates the light beam in the direction of the grating 13, and a focusing lens 16 focuses the dispersed spectral components to the line sensor 14.

The reference path 6 is of bidirectional type, i.e. the reference light is reflected in the reference path to return in opposite direction to the point at which it entered the reference path. In the shown embodiment, this is achieved with a mirror 17 at the terminating point of the reference path. The mirror 17 is pivotable. A driving mechanism (not shown), e. g. a galvanometric drive, is provided to pivot the mirror 17 from a reflecting position as shown in box (A) into a deflecting position as shown in box (B) and vice versa. The light in the reference path is interrupted if the mirror 17 is in deflecting position. A collimating lens 18 and a focusing lens 19 are provided for optical alignment. An element of light absorbing material 20 on which the deflected light impinges is provided in order to ensure that no light portion may return the reference path 6.

The driving mechanism for the mirror 17 is controlled by a microprocessor controller 21. This microprocessor controller 21 also controls the non-interferometric detector 12 so that the latter becomes operable when the mirror 17 is in deflecting position. The signal generated by the non-interferometric detector 12 is sent to an analog-to-digital converter card ADC mounted in a personal computer 22 with a display 23. The personal computer 22 further contains a frame grabber card FG which records data from the line sensor 14 for SOCT when the mirror 16 is in reflecting position. The frame grabber card FG is controlled by the microprocessor controller 21, too. The latter also controls the galvanometric scanner mirror 9 and thus the full operation of the system. The microprocessor controller 21 may be integrated in the personal computer 22. In particular, it may be a software system communicating with the controlled devices via USB.

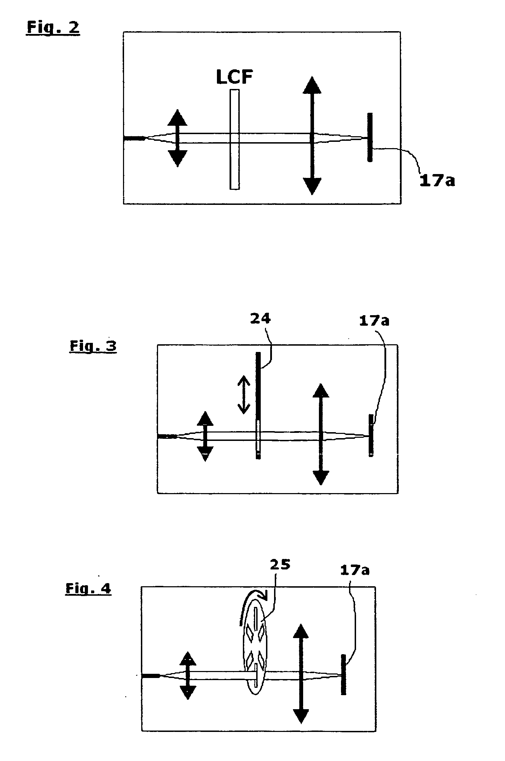

Fig. 2 shows an alternative embodiment for the terminal part of the reference path 6 with a fixed mirror 17a reflecting the light back into the reference path 6. Interruption of the reference light is performed by a liquid crystal filter (LCF), the transmissivity of which is also controlled by the microprocessor controller 21.

Fig. 3 shows, as a further alternative, interruption with a blocking plate 24, while Fig. 4 illustrates a further alternative with a rotating chopper 25. Again, the driving mechanisms (not shown) of the blocking plate 24 and the chopper 25 are controlled by said microprocessor controller 21.

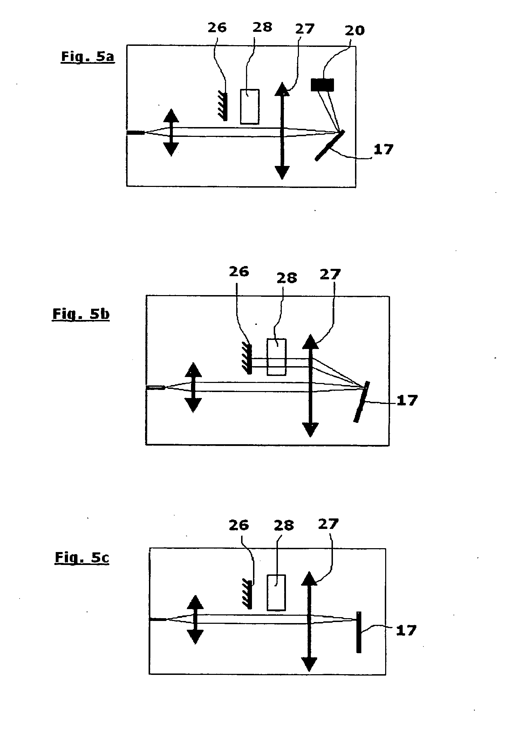

Fig. 5a, 5b and 5c show a particularly advantageous embodiment for the terminal part of the reference path 6. It comprises, next to the pivotable mirror 17, a fixed mirror 26. In certain pivot angles, the pivotable mirror 17 directs the light through a lens 27 to said fixed mirror 26 which retro-reflects it the same way back into the reference path 6, as shown in Fig. 5b. A dispersion compensator 28 is inserted in the path of light between the mirrors 17, 26 to compensate for the dispersion caused by lens 27. Since the light beam impinging on the pivotable mirror 17 is offset from the pivot axis, the length of the path of light changes with the pivot angle of mirror 17. Thus a scannable ODL is achieved. If the pivotable mirror 17 is oriented perpendicularly to the axis of the incident beam, the light is retro-reflected from there back along the reference path 6, as shown in Fig. 5a. In other pivot angles, the pivotable mirror 17 reflects the light in a direction in which it cannot reach the fixed mirror 26 so that it is interrupted. It becomes clear that the scanning device can be used to perform the interruption and no extra components are needed.

It is to be noted that displacing the pivotable mirror 17 along the axis of the incident light yields a fourfold extension of the reference path length. Displacement of ordinary devices such as corner cubes caused the path length only to double. So the pivotable mirror 17 is preferably disposed displaceable along the axis of the incident beam within the apparatus of the invention. A driving mechanism for the displacement is preferably controlled by the microprocessor controller 20.

## Claims

1. Apparatus for optical coherence tomography and non-interferometric imaging of a sample (7) comprising
· illuminating optics (1, 2, 3, 8, 9, 10) arranged for illuminating the sample (7) along an illumination path,
· first splitting optics (5) arranged for splitting off a portion of light from the illumination path and directing said portion into a reference path (6),
· an optical coherence tomography detection device (11),
· a separate second detection device (12) for non-interferometric imaging,
· recombining optics (5) arranged for recombining light from the reference path (6) with light returning from the sample (7) and directing the recombined light along an output path to the optical coherence tomography detection device (11),
· second splitting optics (4) arranged for splitting off a portion of light returning from the sample (7) and directing this split off portion of light to the separate second detection device (12),
· **characterized in that**
· said second splitting optics (4) are disposed along the output path and
· the apparatus further comprises interrupting means (17, 20) arranged for interrupting light in the reference path (6).

2. Apparatus according to claim 1, **characterized in that** the interrupting means is provided by a scannable optical delay line.

3. Apparatus according to claim 2, **characterized in that** the scannable optical delay line comprises a pivotable mirror arranged for deflecting light to a second mirror arranged for directing the light along the reference path to the recombining device.

4. Apparatus according to claim 3, **characterized in that** the pivotable mirror is arranged for pivoting into an angle position in which the deflected light does not reach the reflecting surface of the second mirror.

5. Apparatus according to any of the preceding claims, **characterized in that** it further comprises a triggering device adapted to activate the second detection device in phases in which the light in the reference path is interrupted.

6. Apparatus according to any of the preceding claims, **characterized in that** the recombining optics is identical with the first splitting optics.

7. Apparatus according to any of the preceding claims, **characterized in that** the second splitting optics is identical with the recombining optics.

## Patentansprüche

1. Vorrichtung für optische Kohärenztomographie und nicht-interferometrische Abbildung einer Probe (7) umfassend
• eine Beleuchtungseinrichtung (1, 2, 3, 8, 9, 10), eingerichtet zum Beleuchten der Probe (7) entlang eines Beleuchtungswegs,
• eine erste Strahlteilungseinrichtung (5), eingerichtet zum Abspalten eines Lichtanteils aus dem Beleuchtungsweg und zum Führen dieses Anteils in einen Referenzweg (6),
• eine Messeinrichtung für optische Kohärenztomographie (11),
• eine separate zweite Messeinrichtung (12) für nicht-interferometrische Abbildung,
• eine Überlagerungseinrichtung (5), eingerichtet zum Überlagern von Licht aus dem Referenzweg (6) mit Licht, das von der Probe (7) zurückkehrt, und zum Führen des überlagerten Lichts entlang eines Ausgabewegs zur Messeinrichtung für optische Kohärenztomographie (11),
• eine zweite Strahlteilungseinrichtung (4), eingerichtet zum Abspalten eines Lichtanteils, der von der Probe (7) zurückkehrt, und zum Führen dieses abgespaltenen Lichtanteils zu der separaten zweiten Messeinrichtung (12),
**dadurch gekennzeichnet, dass**
• die zweite Strahlteileinrichtung (4) entlang des Ausgabewegs angeordnet ist und
• die Vorrichtung ferner Unterbrechungsmittel umfasst, eingerichtet zum Unterbrechen des Lichts im Referenzweg (6).

2. Vorrichtung nach Anspruch 1, **dadurch gekennzeichnet, dass** das Unterbrechungsmittel von einer einstellbaren Verzögerungsoptik gebildet ist.

3. Vorrichtung nach Anspruch 2, **dadurch gekennzeichnet, dass** die einstellbare Verzögerungsoptik einen schwenkbaren Spiegel umfasst, eingerichtet zum Ablenken des Lichts zu einen zweiten Spiegel, der eingerichtet ist zum Führen des Lichts entlang des Referenzwegs zur Überlagerungseinrichtung.

4. Vorrichtung nach Anspruch 3, **dadurch gekennzeichnet, dass** der schwenkbare Spiegel eingerichtet ist zum Schwenken in eine Winkelposition, in der das abgelenkte Licht nicht die reflektierende Oberfläche des zweiten Spiegels erreicht.

5. Vorrichtung nach einem der vorstehenden Ansprüche, **dadurch gekennzeichnet, dass** sie weiter eine Auslöseeinrichtung umfasst, die zum Aktivieren der zweiten Messeinrichtung in Phasen, in denen das Licht im Referenzweg unterbrochen ist, eingerichtet ist.

6. Vorrichtung nach einem vorstehenden Ansprüche, **dadurch gekennzeichnet, dass** die Überlagerungseinrichtung mit der ersten Strahlteilungseinrichtung identisch ist.

7. Vorrichtung nach einem der vorstehenden Ansprüche, **dadurch gekennzeichnet, dass** die zweite Strahlteilungseinrichtung identisch mit der Überlagerungseinrichtung ist.

## Revendications

1. Appareil de tomographie en cohérence optique et d'imagerie non interférométrique d'un échantillon (7) comprenant :
des dispositifs optiques d'éclairage (1, 2, 3, 8, 9, 10) agencés pour éclairer l'échantillon (7) le long d'un chemin d'éclairage ;
un premier dispositif optique de séparation (5), agencé pour séparer une partie de la lumière du chemin d'éclairage et diriger ladite partie dans un chemin de référence (6) ;
un dispositif de détection par tomographie en cohérence optique (11) ;
un dispositif de détection secondaire séparé (12) d'imagerie non interférométrique ;
un dispositif optique de recombinaison (5), agencé pour recombiner la lumière provenant du chemin de référence (6) avec la lumière revenant de l'échantillon (7) et diriger la lumière recombinée le long d'un chemin de sortie vers le dispositif de détection par tomographie en cohérence optique (11) ;
un second dispositif optique de séparation (4), agencé pour séparer une partie de la lumière revenant de l'échantillon (7) et diriger cette partie de lumière séparée vers le dispositif de détection secondaire séparé (12) ;
**caractérisé en ce que** :
ledit second dispositif optique de séparation (4) est disposé le long du chemin de sortie ; et
l'appareil comprend en outre des moyens d'interruption (17, 20) agencés pour interrompre la lumière dans le chemin de référence (6).

2. Appareil selon la revendication 1, **caractérisé en ce que** les moyens d'interruption sont constitués par une ligne à retard optique réglable.

3. Appareil selon la revendication 2, **caractérisé en ce que** la ligne à retard optique réglable comprend un miroir pivotant agencé pour dévier la lumière vers un second miroir agencé pour diriger la lumière le long du chemin de référence vers le dispositif de recombinaison.

4. Appareil selon la revendication 3, **caractérisé en ce que** le miroir pivotant est agencé pour pivoter dans une position angulaire dans laquelle la lumière déviée n'atteint pas la surface réfléchissante du second miroir.

5. Appareil selon l'une quelconque des revendications précédentes, **caractérisé en ce qu'**il comprend en outre un dispositif de déclenchement adapté pour activer le second dispositif de détection dans les phases pendant lesquelles la lumière est interrompue dans le chemin de référence.

6. Appareil selon l'une quelconque des revendications précédentes, **caractérisé en ce que** le dispositif optique de recombinaison est identique au premier dispositif optique de séparation.

7. Appareil selon l'une quelconque des revendications précédentes, **caractérisé en ce que** le second dispositif optique de séparation est identique au dispositif optique de recombinaison. ,
